# EUROPEAN PATENT APPLICATION

(11) **EP 4 357 460 A1**
(43) Date of publication of application: **24.04.2024**
(21) Application number: 22383014.2
(22) Date of filing: 21.10.2022
(51) Int. Cl.: C12P 7/42, C12P 7/44, C12P 13/00, C12N 9/18, A01N 63/50

(54) **FUMONISIN ENZYMATIC DEGRADATION**

(71) Applicant: Consejo Superior De Investigaciones Científicas, 28006 Madrid (ES); Patent Co Doo., 24 211 Misicevo (RS)
(72) Inventor: Ferrer Martínez, Manuel, 28049 Madrid (ES); Almendral Nieto, David, 28049 Madrid (ES); Raj, Jog, 24 221 Misicevo (RS); Farka , Hunor, 24 221 Misicevo (RS)
(74) Representative: Pons

(57) **Abstract**

The present invention relates to the use of an enzyme which comprises an amino acid sequence with, at least, a 80% sequence identity to sequence SEQ ID NO: 1, for fumonisin degradation in a sample. Furthermore, the present invention relates to a method for fumonisin degradation, based on the incubation of the contaminated sample with an enzyme comprising an amino acid sequence with, at least, a 80% sequence identity to sequence SEQ ID NO: 1.

## Description

The invention belongs to the field of agri-food and biotechnology. Particularly, the present invention describes the use of an enzyme for fumonisin degradation and a method thereof.

### BACKGROUND ART

Many species of fungi capable of growing in grain and animal feed produce secondary metabolites called mycotoxins. Mycotoxins are the biggest challenge for animal feed producers and therefore, regular monitoring and remediation is necessary, as these toxins can be very detrimental to both humans and animals.

Fumonisins are a group of mycotoxins present ubiquitously in nature, which pose detrimental health hazards on humans as well as on animals since they contaminate several food and feed matrices. In fact, the economic loss in the agri-food sector is estimated to amount to billions of dollars, so the economic potential of protection or fumonisin removal measures is correspondingly high.

There are already different measures available today, such as mechanical, thermic, or chemical approaches. However, these kinds of approaches have as an associated disadvantage the possibility of unknown or unintended side products generation which may turn out as dangerous as the original toxin.

In this context, enzymatic detoxification is currently a very promising subject of research and development, as enzymes can remove the mycotoxins in a defined way. Some documents in the state of the art disclose fumonisin enzymatic degradation:
An enzyme degrading Fumonisin B1 toxin, FumD, is disclosed in WO2010031101A1. This document describes an additive for the enzymatic degradation of fumonisins in vegetable raw materials and mixtures containing vegetable raw materials, characterized in that it contains an enzyme with an amino acid sequence corresponding to FB1-degrading FumD hydrolase.

In Heinl S, et al., J Biotechnol. 2010 Jan 15;145(2):120-9, two genes of *Sphingopyxis* sp. MTA144, *fumD* and *fuml,* whose gene products degrade mycotoxin FB1 by their consecutive active, are reported. In particular, it is described their isolation, expression at small scale and enzymatic activity.

Nevertheless, given the important implications that fumonisin degradation have in the agri-food industry and the few enzymatic fumonisin detoxification methods known in the state of the art, alternative enzymatic fumonisin degradation approaches are needed.

### DESCRIPTION OF THE INVENTION

The present invention describes the use of an enzyme which comprises an amino acid sequence with, at least 80%, sequence identity to sequence SEQ ID NO: 1, in the enzymatic degradation of a group of mycotoxins, fumonisins, and a method for fumonisin degradation based on the incubation of the sample of interest and the mentioned enzyme.

The inventors have demonstrated that this enzyme, also referred in the present document as "FB1 hydr", was able to degrade fumonisin B1 (FB1) at different concentrations (Table 4 and 5), in a fast way (Table 7), and being able to retain its capacity to degrade fumonisins after its incubation at different temperatures and at different pH, as observed in the thermostability tests (Table 8) and pH stability tests (Table 9), respectively. Furthermore, fumonisin degradation was achieved in different kind of samples, such as in naturally contaminated corn samples (Table 10) and in gastric and intestinal juice samples (Table 13). In addition, FB1 hydr enzyme efficiently degrades, not only FB1, but also fumonisin B2 (FB2) and fumonisin B3 (FB3) (Table 14).

Based on these results, the invention shows clear industrial applications, since mycotoxins represents a challenge in the agro-alimentary sector. In fact, the use of FB1 hydr in fumonisin degradation shows different advantages. Compared to other approaches, enzymatic degradation of fumonisins avoid or reduce the possibility of unknown or unintended side products generation, which may turn out as dangerous as the original mycotoxin, given that the enzyme targets specific toxins, removing them in a defined way. Moreover, fumonisin degradation is performed at low doses of the enzyme and in a fast way. Additionally, FB1 hydr has thermostability and pH stability associated properties, retaining the capacity to degrade fumonisins in different conditions.

Thus, an aspect of the invention relates to the use of an enzyme which comprises an amino acid sequence with, at least, 80% sequence identity to sequence SEQ ID NO: 1, for fumonisin degradation in a sample, hereinafter the "use of the invention".

The phrase "fumonisin degradation", as used herein refers to the enzymatic modification of one or more fumonisins molecules which causes a decrease or loss of the concentration and/or activity of the fumonisin/s molecule/s. Methods and techniques to assess compounds/metabolites degradation, in the present invention fumonisin/s molecules, are known in the state of the art, such as, but without limiting to, mass spectrometry (MS) techniques.

The term "fumomisin", also abbreviated as "FB", refers to a group of mycotoxins produced by pathogenic fungi. Chemically, fumonisins are characterized by having two methyls (-CH₃), one amine (-NH₂), one to four hydroxyl (-OH⁻), and two tricarboxylic ester groups located at different positions along with the linear polyketide-derived backbone.

More than 15 fumonisin homologues have been known and characterized as fumonisin A, B, C, and P. Further among fumonisin B, FB1, FB2, and FB3 are most abundant with FB1 being the most toxic form that can co-exists with other forms of fumonisin, i.e., FB2 and FB3. These (FB1, FB2, and FB3) forms are the main food contaminants.

In the present invention, the enzyme used for fumonisin degradation, also referred in the present document as "FB1 hydr" (or simply "FB1 enzyme"), and which may also be referred as the "enzyme of the invention" hereinafter, comprises an amino acid sequence with, at least, 80% sequence identity to sequence SEQ ID NO: 1.

SEQ ID NO: 1
MSTARLTPTSASTLKLLSVAVAALTLSACLSGGGGGGADGPNPLAIETSEGKVVGI
SNDGIRVFRGIPYAAPPVGDLRLAPPQPPASRSETLRLSEEFGNSCPQSDLTTGQQ
VGNEDCLYLNVYAPAEAEDLPVMVWIHGGAFVFGNGGGEYDPTRLVEQDVIVVTL
NYRLGNLGFLAHPALESDAGNFALMDQQLALAWVKENITAFGGDPANVTIFGESA
GGHSVMSHIVSPRAEEADLFQRAIVQSGSYAPFQMPKATAQFLGTSVANGLGCTD
PETAASCLRSLPVSAFLAAQGSQSIPVVDPDDDLLPKSIQQALADGDFNSSLDIMIG
SNQNEGTLFVALDEVGGDPIDGEAEYRERVAEFFQPYQASIPFDDDQIATDYLDFV
DGEAKPFEAALSGIWTDFMFACNAYSQASTFAGASMNTFQYWFRDEDAPWTLVP
PFAV

A preferred embodiment provides the use of the invention wherein the enzyme comprises an amino acid sequence with a sequence identity of, at least, 85, 90, 91, 92, 93, 94, 95, 96, 97, 98 or 99% to SEQ ID NO: 1. More preferably, the enzyme comprises, or consists of, the amino acid sequence SEQ ID NO: 1.

A still more preferred embodiment provides the use of the invention, wherein the enzyme comprises an amino acid sequence with a sequence identity of, at least, 80, 85, 90, 91, 92, 93, 94, 95, 96, 97, 98 or 99% to SEQ ID NO: 2. Even more preferably, the enzyme comprises, or consists of, the amino acid sequence SEQ ID NO: 2.

SEQ ID NO: 2
MAHHHHHHVGTGSNDDDDKSPDPMSTARLTPTSASTLKLLSVAVAALTLSACLSG
GGGGGADGPNPLAIETSEGKVVGISNDGIRVFRGIPYAAPPVGDLRLAPPQPPASR
SETLRLSEEFGNSCPQSDLTTGQQVGNEDCLYLNVYAPAEAEDLPVMVWIHGGAF
VFGNGGGEYDPTRLVEQDVIVVTLNYRLGNLGFLAHPALESDAGNFALMDQQLAL
AWVKENITAFGGDPANVTIFGESAGGHSVMSHIVSPRAEEADLFQRAIVQSGSYAP
FQMPKATAQFLGTSVANGLGCTDPETAASCLRSLPVSAFLAAQGSQSIPVVDPDD
DLLPKSIQQALADGDFNSSLDIMIGSNQNEGTLFVALDEVGGDPIDGEAEYRERVA
EFFQPYQASIPFDDDQIATDYLDFVDGEAKPFEAALSGIWTDFMFACNAYSQASTF
AGASMNTFQYWFRDEDAPWTLVPPFAVSFPLGATHAGEIPYVLYPQAIMEQRYTG
DPDDLNSLAGEMVDYWTQFAKTGDPNTTDGVAAAWQQAATGNLMTLDVPNASN
ANTLGFLGYHHCSYWADPPLVLR

As it can be seen, SEQ ID NO: 2 is an amino acid sequence comprising SEQ ID NO: 1.

In the present invention, "sequence identity" means the degree of similarity between two nucleotide or amino acid sequences obtained by aligning the two sequences. Depending on the number of common residues between the aligned sequences, a degree of identity expressed as a percentage will be obtained. The degree of identity between two nucleotide or amino acid sequences can be determined by conventional methods, e.g. by standard sequence alignment algorithms known in the state of the art, such as BLAST [Altschul S.F. et al. Basic local alignment search tool. J Mol Biol. 1990 Oct 5; 215(3):403-10]. BLAST programs, e.g. BLASTN, BLASTX, and TBLASTX, BLASTP and TBLASTN, are in the public domain on the website of The National Center for Biotechonology Information (NCBI).

The enzyme of the invention can be obtained by techniques widely known in the prior art. Examples of techniques for obtaining proteins, such as the enzyme of the invention, include, without limitation to, chemical or biological synthesis, genetic recombination or expression of polynucleotides coding for the enzyme of the invention.

As shown later on in the present document, the inventors have demonstrated that FB1 hydr efficiently degrades toxins others than FB1, in particular FB2 and FB3. Thus, in a preferred embodiment of the use of the invention, alone or in combination with the rest of preferred embodiments of the invention, the fumonisin degradation comprises the degradation of at least one fumonisin selected from the list consisting of FB1, FB2, FB3 and any combination thereof. More preferably, the fumonisin is FB1.

FB1, FB2 and FB3 are different types of fumonisins, whose chemical structures are summarized below:

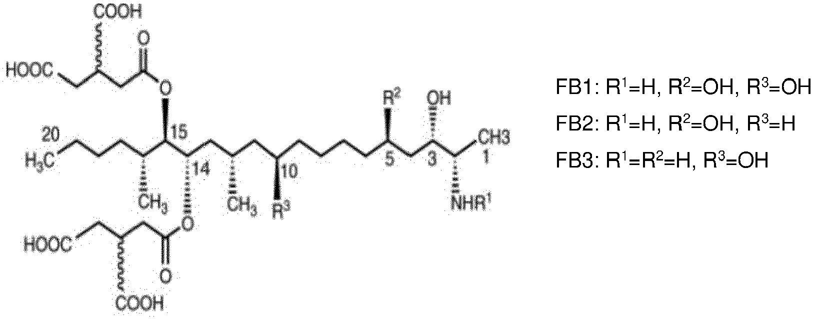

In another preferred embodiment of the use of the invention, alone or in combination with the rest of the preferred embodiments, fumonisin concentration is from 1 to 25 ppm (parts per million). More preferably, fumonisin concentration is from 2 to 20 ppm. Even more preferably, fumonisin concentration is selected from the list consisting of 2, 3, 4, 5, 6, 7, 8, 9, 10, 11, 12, 13, 14, 15, 16, 17, 18, 19 and 20 ppm.

Besides, any sample contaminated with, or where fumonisins can be generated, or any sample susceptible to be contaminated with them is suitable for the use of the invention. However, in a preferred embodiment of the use of the invention, alone or in combination with the rest of the preferred embodiments, the sample is a raw material sample or a mixture comprising raw material.

The term "raw material" refers to any resource found in nature and unprocessed or minimally processed materials, including vegetable raw materials.

In a more preferred embodiment of the invention, the raw material is a vegetable raw material.

Vegetable raw materials are raw materials with a vegetable origin. Examples of vegetable raw materials include, without limitation to, cereals, grasses, fruits, vegetables and intermediate products containing these substances for the production of foods and feeds such as corn, wheat and distilled grains.

In an even more preferred embodiment of the use of the invention, the vegetable raw material is selected from the list consisting of corn, wheat and distilled grains.

In another preferred embodiment of the use of the invention, alone or in combination with the rest of preferred embodiments, the sample is food or a feed product.

As used herein, the terms "food" and "feed product" are equivalent and can be used indistinctly throughout the present description. The food product can be in liquid, powdery, or solid form. The food may be for human or non-human animal consumption, including feed and animal food. Foods comprise functional foods commonly known in the art. Examples of foods include, but are not limited to, feed, dairy products, vegetable products, meat products, snacks, confectionery products, fodder, drinks, baby food, cereals, fried foods, industrial bakery products and biscuits. Examples of dairy products include, but are not limited to, products derived from fermented milk (for example, but not limited to, yogurt or cheese) or non-fermented milk (for example, but not limited to, ice cream, butter, margarine or whey). The vegetable product is, for example, but not limited to, a cereal in any form of presentation. In the present document, by food and feed product is also meant a part or small quantity isolated from them.

In another preferred embodiment of the use of the invention, alone or in combination with the rest of preferred embodiments, the sample is an isolated biological sample. Preferably, the isolated biological sample is intestinal and/or gastric juice. More preferably, the isolated biological sample is pig intestinal juice and/or pig gastric juice.

The term "isolated biological sample", refers to any sample isolated from a subject and includes, but is not limited to, biological fluids from an individual, obtained by any method known to a person skilled in the art for that purpose.

Inventors have used the enzyme of the invention at different concentrations in efficient fumonisin degradation. A preferred embodiment provides the use of the invention, wherein the enzyme of the invention concentration is from 2.5 µg/mL to 10000 µg/mL (including the end values of the range). A more preferred embodiment, provides the use of the invention, wherein the enzyme of the invention concentration is from 5 µg/mL to 1000 µg/mL (including the end values of the range). Even more preferably, the enzyme of the invention is at a concentration selected from the list consisting of: 5, 10, 20, 25, 30, 45, 50, 55, 60, 65, 70, 75, 80, 85, 90, 95, 100, 150, 200, 250, 300, 350, 400, 450, 500, 550, 600, 650, 700, 750, 800, 900, 950, and 1000 µg/mL.

Furthermore, the inventors have performed fumonisin degradation tests at different pH. A preferred embodiment, alone or in combination with the rest of preferred embodiments, provides the use of the invention, wherein the pH is between 5 to 8 (including the end values of the range), more preferably wherein the pH is 6.0-8.0.

As it has been already mentioned in the present document, application of the enzyme of the invention in a sample has demonstrated efficient fumonisin degradation. Inventors of the present invention have also developed a method for fumonisin degradation.

Thus, a second aspect of the present invention relates to a method for fumonisin degradation in a sample, hereinafter the "method of the invention", which comprises the following step:
a) incubating the sample with an enzyme which comprises an amino acid sequence with, at least, 80% sequence identity to sequence SEQ ID NO: 1.

A preferred embodiment provides the method of the invention wherein the enzyme comprises an amino acid sequence with a sequence identity of, at least, 85, 90, 91, 92, 93, 94, 95, 96, 97, 98 or 99% to SEQ ID NO: 1. More preferably, the enzyme of the invention comprises, or consists of, the amino acid sequence SEQ ID NO: 1.

A still more preferred embodiment provides the method of the invention wherein the enzyme comprises an amino acid sequence with a sequence identity of, at least, 80, 85, 90, 91, 92, 93, 94, 95, 96, 97, 98 or 99% to SEQ ID NO: 2. Even more preferably, the enzyme comprises, or consists of, the amino acid sequence SEQ ID NO: 2.

The phrase "incubating the sample with an enzyme", as used herein, refers to put in contact the enzyme of the invention, in a reaction mixture. As it is known by a person skilled in the art, a reaction mixture can include other reagents and/or buffers known in the state of the art. In a preferred embodiment of the method of the invention, alone or in combination with the rest of preferred embodiments, the step (a) comprises incubating the sample with the enzyme of the invention in phosphate buffer, preferably, at a concentration of 10-100 mM (including the end values of the range).

As previously mentioned in the present document, inventors have used the enzyme of the invention at different concentrations in efficient fumonisin degradation. Thus, a preferred embodiment provides the method of the invention wherein the enzyme of the invention concentration is from 2.5 µg/mL to 10000 µg/mL (including the end values of the range). A more preferred embodiment provides the method of the invention, wherein the enzyme of the invention concentration is from 5 µg/mL to 1000 µg/mL (including the end values of the range). Even more preferably, the enzyme of the invention is at a concentration selected from the list consisting of: 5, 10, 20, 25, 30, 45, 50, 55, 60, 65, 70, 75, 80, 85, 90, 95, 100, 150, 200, 250, 300, 350, 400, 450, 500, 550, 600, 650, 700, 750, 800, 900, 950, and 1000 µg/mL.

Furthermore, the step (a) may be performed under certain incubation conditions. In a preferred embodiment of the method of the invention, the step (a) comprises incubating the sample with the enzyme of the invention during 2.5 min to 24 hours (including the end values of the range). Preferably, the step (a) comprises incubating the sample with the enzyme of the invention during 2.5 min to 90 min (including the end values of the range). More preferably, the step (a) comprises incubating the sample with the enzyme of the invention during 5 to 30 min (including the end values of the range). Even more preferably, the step (a) comprises incubating the sample with the enzyme of the invention during a period of time selected from the list consisting of 5, 10, 15, 20, 25 and 30 min.

In another preferred embodiment of the method invention, alone or in combination with the rest of preferred embodiments, the step (a) comprises incubating the sample with the enzyme of the invention at a temperature between 30ºC to 90ºC (including the end values of the range). More preferably, the step (a) comprises incubating the sample with the enzyme of the enzyme of the invention at a temperature selected from the list consisting of 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, and 90 ºC.

In another preferred embodiment of the method of the invention, alone or in combination with the rest of preferred embodiments, the step (a) comprises incubating the sample with the enzyme of the invention at pH 5 to pH 8 (including the end values of the range). Preferably, the step (a) comprises incubating the sample with the enzyme of the invention at a pH selected from the list consisting of pH 5, pH 6, pH 7 and pH 8.

In a still more preferred embodiment of the method of the invention, the step (a) comprises incubating the sample with the enzyme of the invention during 5 min to 90 min (including the end values of the range) at a temperature between 37ºC to 90ºC (including the end values of the range) and at pH 5 to pH 8 (including the end values of the range).

As mentioned in the previous aspect of the invention, the inventors have demonstrated that FB1 hydr efficiently degrades toxins other than FB1, in particular FB2 and FB3. Thus, in another preferred embodiment of the method of the invention, alone or in combination with the rest of preferred embodiments, the fumonisin degradation comprises the degradation of at least one fumonisin selected from the list consisting of FB1, FB2, FB3 and any combination thereof. More preferably, the fumonisin is FB1.

In another preferred embodiment of the method of the invention, alone or in combination with the rest of the preferred embodiments, fumonisin concentration is from 1 to 25 ppm (parts per million). More preferably, fumonisin concentration is from 2 to 20 ppm. Even more preferably, fumonisin concentration is selected from the list consisting of 2, 3, 4, 5, 6, 7, 8, 9, 10, 11, 12, 13, 14, 15, 16, 17, 18, 19 and 20 ppm.

Moreover, as it has been also mentioned in the previous aspect of the invention, the enzyme of the invention efficiently degrades fumonisins in different kinds of samples.

In a preferred embodiment of the method of the invention, alone or in combination with the rest of the preferred embodiments of the method of the invention, the sample is a raw material sample or a mixture comprising raw material. In a more preferred embodiment of the method of the invention, the raw material is a vegetable raw material.

In an even more preferred embodiment of the method of the invention, the vegetable raw material is selected from the list consisting of corn, wheat and distilled grains.

In another preferred embodiment of the method of the invention, the sample is food or a feed product.

In another preferred embodiment of the method of the invention, alone or in combination with the rest of preferred embodiments, the sample is an isolated biological sample. Preferably, the isolated biological sample is intestinal and/or gastric juice. More preferably, the isolated biological sample is pig intestinal juice and/or pig gastric juice.

The terms used to define the method of the invention have been described in the previous aspect of the invention, and apply, as well as their preferred embodiments, to the method of the invention.

### Examples

### 1. Materials and methods

### 1.1. Enzyme source, production and purification

The sequence encoding FB1 hydr enzyme (SEQ ID NO: 4) was selected by DIAMOND search tool, using the sequence of FumD (SEQ ID NO: 3) as target for search, and sequence repositories (in house and public). For DIAMOND searches default parameters were used (percent identity >60%; alignment length >70; e-value < 1e⁻⁵). The identified sequence encoding FB1hydr (SEQ ID NO: 4) was used as template for gene synthesis, and gene was codon-optimized to maximize expression in *Escherichia coli* (*E. coli*)*.*
SEQ ID NO: 3 
MKEHQCRGGRASPAAPATWLARISVSRGASAIAWTFMLGATAIPVAAQTDDPKLV
RHTQSGAVEGVEGDVETFLGIPFAAPPVGDLRWRPPAPPRAWAGTRDGRRFAPD
CIGNERLREGSRAAGTSEDCLYLNIWSPKQVGKGGLPVMIWVYGGGFSGGSGAV
PYYDGSALAQKGVVVVTFNYRAGILGFLAHPALSKESPNGVSGNYGLLDMLAAFK
WVQNNIREFGGDPNRVTVFGESAGASALGLLLTSPLSESAFNQAILQSPGLARPLA
TLSESEANGLELGADISALRRADAGLTKIAQSRIPMSRQFTKPRPMGPILDGYVLRT
LDVDAFAKGAFRKIPVLVGGNADEGRAFTDRLPVKTVLEYRAYLTEQFGDEADAW
ERCYPANSDADVPAAVARLFGDSQFNNGIELLSAAFAKWRTPLWRYRFTGIPGAG
RRPATHGDEIPYVFANLGPSSVSMFGSLEGGAGASDIKLATEMSAAWVSFAVHGV
PDQGTKSHWPRFERRGEIMTFGSQVGSGEGLGVSPSKACQPSK
SEQ ID NO: 4

ATGCTGCTGAGCGGCCGTACCTTCGCGCTGGTGTTTGGTGCGCCGCTGCTGG
GTGCGCTGGCGGCGTTCGCGGTGCCGGTTGCGGCGGCGAGCGGTCGTCCGA
CCGCGACCGTGGCGGAAGGTCGTGTTGCGGGCAGCCTGGAGGACGGTGTGC
GTGTTTTTAAAGGTCTGCCGTATGCGCTGCCGCCGGTGGGTGTTCGTCGTTGG
CGTCCGCCGGCTCCGCCGGCGAGCTGGCAAGGTGCGCGTGAAGCGCATGCG
TTTGGTCCGGATTGCGTGCAACCGCCGTACCCGGCGGACAGCGTTTATTTTGA
ACCGCCGCGTCCGACCAGCGAGGATTGCCTGACCCTGAACGTTTGGGCGCCG
CGTAGCGCGGCGCGTGCGCCGGTGATTGTTTGGATTCACGGTGGCGCGCTGC
AGCATGGTGGCAGCGCGAGCCCGATGTATGACGGTCGTGAGTATGCGAAACG
TGGCATCGTGTTCGTTAGCATTAACTACCGTCTGGGTATCTTTGGTTGGCTGGC
GCACCCGGGTCTGAGCGCGGAAGCGCCGGATCGTGTGAGCGGTAACTATGGT
CTGCTGGACCAGATTGCGGCGCTGGAGTGGGTTAAGCGTAACATTGCGGCGT
TTGGTGGCGATCCGGCGAACGTGACCGCGATGGGTGAAAGCGCGGGTGCGCT
GAGCGTTACCTACCTGCTGGCGGCGCCGCGTGCGAAAGGTCTGTTTGGCCGT
GCGATCGTGCAAAGCACCAACATGCGTGCGGTTCCGCGTCTGCGTGACCCGG
CGTATGGTCTGCCGAGCGCGGAAGCGATCGGTGCGGCGTTTGCGGAGAGCGT
GGGTGCGAAGAACCCGGCGGCGCTGCGTGCGCTGGATGCGGAGGCGCTGAC
CGCGGCGGCGCTGCGCGCGCGCTTTGCGGCGCAGCCGACCATTGATGGTGC

GCTGCTGCCGGCGCAGATTGTGGATGTTTTCGACCGTGGTGAACAAATCAAAG
TGCCGGTTCTGGCGGGTTTTAACAGCGGCGAGGTGCGCAGCCAACGTGGTCT
GGTGCCGCCGGTTCCGGCGGATGCGGCGGCGTATGCGGCGGGTGCGGCGC
GTCGTTATGGTGACCTGGCGCCGGCGTTCCTGCGTCTGTACCCGGCGAGCGA
TATGGAGGAAAGCCTGATCGCGGCGACCCGTGACATTGTTTATGGCTGGGCG
GTGGAACGTCTGGTTCGTCAGCAAACCGTGGCGGGTGTTCCGAGCTACCTGTA
CCTGTTCGATCACTGCTATGATGCGGCGCGTGCGCGTAAGCTGTGCGCGTTTC
ACGCGAGCGAGATCCCGTTCATGTTTGGTAGCATTGGCCGTGCGGTGCCGCA
GGCGGCGAACTGGCCGGCGGCGACCGGTGATGATGACCGTCGTCTGGCGCG
TCAAATGGTGGATTATTGGAGCAGCTTCGCGGCGCGTGGTATCCCGAGCGCG
GTTGGTGCGCCGGACTGGCGTCCGTACGGCGAGGAAGAGTATTTTCTGGAAC
TGGGTGCGAGCCCGGTTCTGCGTCGTGATCCGATGCCGGGCATGTTCGAGTT
TCGTGAAGAGATTATGCGTCGTCAGCGTCAACGTGGCGAACAGTGGTTCGTGA
ACACCGGTCCGGTTGACCGTGAG

The genes was flanked by BamHI and Hindlll (stop codon) restriction sites and delivered in pET-45b(+) expression vector with ampicillin selection marker (GenScript, US). This plasmid, introduced in *E. coli* BL21(DE3), supports the expression of N-terminal histidine (his) fusion proteins. The soluble His-tagged proteins were produced and purified at 4°C after binding to a Ni-NTA His-Bind resin (Merck Life Science S.L.U., Madrid, Spain). The purity was assessed as >98% using SDS-PAGE analysis in a Mini PROTEAN electrophoresis system (Bio-Rad, Madrid, Spain). Purified protein was stored at -86°C until use at a concentration of 10 mg mL⁻¹ in 40 mM 4-(2-hydroxyethyl)-1-piperazineethanesulfonic acid (HEPES) buffer (pH 7.0). FB1hydr has been also synthesized (with optimal codon usage for yeast) in the vector pPIC9, that supports the expression of N-terminal histidine (his) fusion proteins, and *Pichia pastoris* (*P. pastoris*), using methanol as inductor. The pPIC9 vector in which the enzyme gene is inserted has a functional HIS4 gene and the *Pichia pastoris* strain GS115 (phenotype Mut+) has that gene deficient (his4). Expression yields in *E. coli* and *P. pastoris* are given in Table 1.

**Table 1. Expression yield of FB1hydr.**

| Vector | Host | Yield (grams/L) |
|---|---|---|
| pET-45b(+) | *E. coli* BL21 (DE3) | 1 gram per liter culture |
| pPIC9 | *P. pastoris* | 0.05 gram per liter culture |

The detailed protocol for expressing the FB1 degrading His₆-tag enzymes in pET-45b(+) and the host *E. coli* BL21 (DE3), is detailed below:
- One colony is picked and used to inoculate 50 mL of Luria Bertani (LB) broth plus antibiotic (ampicillin [Amp] 50 µg/mL) in a 0.1-0.25-I flask. Instead of LB medium, Terrific Broth medium can be used.
- The cultures were then incubated at 37 °C and 150 rpm until an OD between 0.8 -1 (OD at 600 nm).
- The next day, this pre-inoculum was added to a 2.5 L-flask with 1 liter of LB + Amp 50 µg/mL and let it grow at 37ºC and 150 rpm until an optical density (OD) between 0.8 -1 (OD is measured at 600 nm).
- Induction of the culture. The induction method depends on the plasmid. In the case of enzymes for FB1 degradation, the plasmid is pET45b(+) and 1 mM IPTG (Isopropyl β-D-1-thiogalactopyranoside) is used. Leave the culture inducing overnight at 16°C and 200 rpm (EH730 is also available in the plasmid pET45b(+)). The standard expression is done at 16ºC, but the enzyme can be well produced at 30-37ºC.
- Centrifugation of the cultures at 8000 rpm for 5 minutes at 4ºC.
- The pellet was resuspended in 20 mL of HEPES 40 mM pH 7 to wash the pellet, centrifuged at 8000 rpm 5 minutes and at 4ºC.
- The pellet was resuspended in 20 mL of 40 mM HEPES and then freeze dried. Approximately 1 gram of lyophilisate is obtained per liter of culture. The proteins can be also purified using HIS-Select Nickel Affinity Gel.

The detailed protocol for expressing the FB1 degrading His₆-tag enzyme in pPIC9 and the host *P. pastoris* GS115, is detailed below:
- Inoculate one colony in 25 mL BMG (Buffered Minimal Glicerol) in a 250 mL flask (optional: slit flask). Grow at 28-30°C at 200-250 rpm to OD600 = 4-8.
- Collect cells by centrifugation at 1500g.
- Resuspend cells in BMM medium to OD600 = 1 (100-200 mL BMM (Buffered Minimal Methanol) medium) in 1 liter flask preferably with slits.
- Methanol is added to a final concentration of 0.5% upon transfer to BMM medium and every 24 hours thereafter.
- To see maximum protein expression, aliquots are collected at 0, 6, 12, 24 hours and then every 12 hours (ideally, but collecting 2 aliquots each day is sufficient, e.g. one in the morning and one in the evening).
- When best expression conditions are decided, then, separate the cells from the supernatant by centrifugation at 1500 g and then take the supernatant containing the enzyme. The supernatant can be directly liophilized or precipitate, or purified using HIS-Select Nickel Affinity Gel as the protein contain 6-His tags at the C-terminal.

### 1.2. Preparation of FB1hydr samples for degradation tests

Three different types of samples were produced to test their capacity to degrade FB1 toxin.
- The first one refer to a sample consisting in purified FB1hydr, herein named FB1 hydr (pure). To prepare these samples, the soluble His-tagged protein was produced and purified at room temperature after binding to a nickel-nitrilotriacetic acid (Ni-NTA) His-Bind resin (from Merck Life Science S.L.U., Madrid, Spain). Briefly, a single colony of *E. coli,* previously grown at 37 °C on solid Luria Bertani (LB) agar medium supplemented with 100 µg/mL ampicillin (Amp), was picked and used to inoculate 50 mL of LB-Amp medium in a 0.25-liter flask, following by cultivation at 37°C and 150 rpm overnight. Afterwards, 50 mL of this culture were used to inoculate 1-liter of LB-Amp medium in a 2.5-liter flask, which was then incubated at 37 °C to an OD600nm ranging from 0.7 to 0.9. Protein expression was induced by adding IPTG to a final concentration of approx. 1 mM, followed by incubation for 16 h at 16°C. The cells were harvested by centrifugation at 8000 g for 10 min to yield a pellet of 2-3 g per litre (wet weight). The wet cell pellet was re-suspended in 15 mL of 50 mM sodium phosphate, pH 8.0, 10 mM imidazole and 300 mM NaCl. The, 15 µL of Lysonase Bioprocessing Reagent (Novagen, Darmstadt, Germany) was added and incubated for 60 min on ice with rotating mixing. The cell suspension was sonicated for a total of 5 min and centrifuged at 15000 x g for 15 min at 4°C, and the supernatant was retained. The soluble His-tagged protein was purified at 4°C after binding to a HIS-Select^{®} Nickel Affinity Gel (Merck Life Science S.L.U., Madrid, Spain) and eluted following the correspondent protocol. Briefly, once the supernatant flowed through the column, the resin was washed thrice with 50 mM sodium phosphate buffer pH 8 with 0.3 M NaCl and 10 mM imidazole to remove the proteins that did not bind the Ni-resin. At last, the His-tagged protein was eluted using 50 mM sodium phosphate buffer pH 8 with 0.3 M NaCl and 250 mM imidazole. The purified protein solution was concentrated by ultrafiltration through low-adsorption hydrophilic 10000 nominal molecular weight limit cutoff membranes (regenerated cellulose, Amicon). An extensive dialysis of protein solutions against 40 mM HEPES buffer (pH 7.0) was then performed using Pur-A-LyzerTM Maxi 1200 dialysis kit (Sigma-Aldrich, MO, US), as follows. 0.5-3 mL concentrated protein solution were dialyzed against 2-liter buffer during 1 hour at room temperature, after which the buffer was changed by another 2-liter buffer and maintained 1 hour more. Then, the buffer was changed, and the dialysis was kept overnight at 4°C. The dialyzed protein solution was recovered and then freeze dried.
- The second one refer to a sample consisting in protein extracts containing FB1 hydr, herein named FB1 hydr (semi-pure). To prepare these samples, the soluble His-tagged protein was produced and purified at room temperature after binding to a nickel-nitrilotriacetic acid (Ni-NTA) His-Bind resin (from Merck Life Science S.L.U., Madrid, Spain). Briefly, a single colony of *E. coli,* previously grown at 37 °C on solid LB agar medium supplemented with 100 µg/mL ampicillin (Amp), was picked and used to inoculate 50 mL of LB-Amp medium in a 0.25-liter flask, following by cultivation at 37°C and 150 rpm overnight. Afterwards, 50 mL of this culture were used to inoculate 1-liter of LB-Amp medium in a 2.5-liter flask, which was then incubated at 37 °C to an OD600nm ranging from 0.7 to 0.9. Protein expression was induced by adding IPTG to a final concentration of approx. 1 mM, followed by incubation for 16 h at 16°C. The cells were harvested by centrifugation at 8000 g for 10 min to yield a pellet of 2-3 g per litre (wet weight). The wet cell pellet was re-suspended in 15 mL of 40 mM 4-(2-hydroxyethyl)-1-piperazineethanesulfonic acid (HEPES) buffer (pH 7.0). The, 15 µl of Lysonase Bioprocessing Reagent (Novagen, Darmstadt, Germany) was added and incubated for 60 min on ice with rotating mixing. The cell suspension was sonicated for a total of 5 min and centrifuged at 15000 x g for 15 min at 4°C, and the supernatant was retained and then freeze dried.
- The third one refer to a sample consisting in FB1 hydr immobilized on Lifetech ECR8806M Purolite resin, herein named FB1hydr (Purolite). To prepare these samples, the soluble His-tagged protein was produced and purified at room temperature after binding to a nickel-nitrilotriacetic acid (Ni-NTA) His-Bind resin (from Merck Life Science S.L.U., Madrid, Spain). Briefly, a single colony of *E. coli,* previously grown at 37 °C on solid LB agar medium supplemented with 100 µg/mL ampicillin (Amp), was picked and used to inoculate 50 mL of LB-Amp medium in a 0.25-liter flask, following by cultivation at 37°C and 150 rpm overnight. Afterwards, 50 mL of this culture were used to inoculate 1-liter of LB-Amp medium in a 2.5-liter flask, which was then incubated at 37 °C to an OD600nm ranging from 0.7 to 0.9. Protein expression was induced by adding IPTG to a final concentration of approx. 1 mM, followed by incubation for 16 h at 16°C. The cells were harvested by centrifugation at 8000 g for 10 min to yield a pellet of 2-3 g per litre (wet weight). The wet cell pellet was re-suspended in 15 mL of 40 mM 4-(2-hydroxyethyl)-1-piperazineethanesulfonic acid (HEPES) buffer (pH 7.0). The, 15 µL of Lysonase Bioprocessing Reagent (Novagen, Darmstadt, Germany) was added and incubated for 60 min on ice with rotating mixing. The cell suspension was sonicated for a total of 5 min and centrifuged at 15000 x g for 15 min at 4°C, and the supernatant was retained. Then 1 gram of Lifetech ECR8806M Purolite resin, and the suspension maintained overnight at room temperature at 150 rpm. Then, the suspension was filtered and the resin was retained and dried under vacuum.

### 1.3. FB1 degradation tests

The different batches prepared for each of the samples' types, FB1 hydr (pure), FB1 hydr (semi-pure) and FB1hydro (Purolite), were tested for their capacity to degrade FB1 toxin. The assay was setup as follows:
- The lyophilized enzyme material is reconstituted in 0.1 M phosphate buffer pH 7.0.
- An aliquot of the enzyme solution is taken and added to a solution containing 2 ppm FB1.
- The solution is incubated at 37ºC for 5 min to 24 hours.
- The degradation of FB1 is evaluated by MS/MS.

### 2. Results

### 2.1. General characteristics of FB1 hydr

The identified sequence, SEQ ID NO: 1, is substantially different to FumD, by meaning of a 30.1% identity at amino acid level. In order to prove the activity of this enzyme, the gene encoding FB1hydr was synthesized and the protein produced and purified.

Degradation tests using different batches of proteins samples (purified, semi-purified and immobilized) confirmed its capacity to degrade FB1. Results revealed that FB1hydr is active against FB1: 2 ppm FB1 was degrade 100% after 5 min reaction time (Table 2).

**Table 2. FB1 degradation efficiency, measured at 37ºC**

| Type of samples | [Protein] (mg/mL) | Reaction time, min | DEG %, FB1 |
|---|---|---|---|
| FB1 hydr (pure) | 24.4 mg/mL | 60 | 100 |
| FB1 hydr (pure) | 1.60 mg/mL | 5 | 100 |
| FB1 hydr (pure)¹ | 5.0 mq/mL | 15 | 100 |
| FB1 hydr (semi-pure) | 100 mg/mL | 5 | 96.2 |
| FB1 hydr (semi-pure) | 100 mg/mL | 5 | 100 |
| FB1 hydr (semi-pure) | 100 mg/mL | 5 | 100 |
| FB1 hydr (semi-pure) | 100 mg/mL | 5 | 100 |
| FB1 hydr (semi-pure) | 260 mg/mL | 15 | 99.9 |
| FB1 hydr (semi-pure) | 260 mg/mL | 15 | 100 |
| FB1hydro (Purolite) | 200 mg/mL | 5 | 100 |
| FB1hydro (Purolite) | 200 mg/mL | 5 | 100 |

When experiment was performed using 10 ppm FB1, 100% degradation after 5 min was found. Reaction mixture (where 10 ppm FB1 was degraded 100%) was screened in full MS/MS scan for possible metabolites. Obtained data was compared to literature data, and possible metabolites were identified: confirmed HFB1 (Hydrolyzed Fumonisin B1), nontoxic FB1 metabolite.

The thermal stability at 90ºC for 3 min was further tested. For that, the enzyme materials in powder form (FB1 - hydr (purolite) and FB1 - hydr (semi-pure)) were incubated 90ºC for 3 min, and then the buffer and FB1 toxin added, and the degradation percentage evaluated. The results summarized in Table 3 revealed that "FB1hydr" enzyme materials, FB1 hydr (semi pure) and purolite, both degrade 2 ppm FB1 in 5 min and are thermostable at 90ºC.

**Table 3. Thermal stability test at 90ºC for 3 min**

| Sample | Protein amount | Reaction time, min | DEG %, FB1 |
|---|---|---|---|
| FB1hydr (Purolite) | 1.0 g | 5 | 100 |
| | | 10 | 100 |
| | | 60 | 100 |
| FB1 - hydr (semi-pure) | 2.44 g | 5 | 100 |
| | | 10 | 100 |
| | | 60 | 100 |

### 2.2. In deep characterization of semi-pure FB1 hydr

### 2.2.1. Enzyme-dose dependent degradation

The degradation efficiency at different doses of FB1 hydr enzyme was further evaluated. As shown in Table 4, when testing enzyme concentrations from 5 to 0.1 mg/mL, we found 0.1 mg/mL (100 µg/mL) of the enzyme giving highest activity. When experiments were performed at low doses of FB1 enzymes, we found 100% degradation at concentrations of enzymes as low as 10 µg/mL after 30 min incubation of 2 ppm FB1 (Table 5).

Dose of FB1 enzyme, conditions:
- 2 ppm FB1
- 30 mins
- pH 7.0

**Table 4. Enzyme-dose degradation efficiency.**

| Enzyme concentration (mg/mL)¹ | Reaction time (min) | DEG %, 2ppm FB1 |
|---|---|---|
| 0.1 | 30 | 100 |
| 0.4 | 30 | 100 |
| 0.8 | 30 | 100 |
| 1.0 | 30 | 100 |
| 5.0 | 30 | 100 |

| | | |
|---|---|---|
| ¹Semi-pure FB1 enzyme was used. | | |

**Table 5. Enzyme-dose degradation efficiency.**

| Enzyme concentration (µg/mL)¹ | Reaction time (min) | DEG %, 2ppm FB1 |
|---|---|---|
| 100.0 | 30 | 100 |
| 50.0 | 30 | 100 |
| 25.0 | 30 | 100 |
| 10.0 | 30 | 100 |
| 5.0 | 30 | 91.8 |

| | | |
|---|---|---|
| ¹Semi-pure FB1 enzyme was used. | | |

### 2.2.2. Toxin-dose dependent degradation

The degradation efficiency at different doses of FB1 toxin was further evaluated. As shown in Table 6, at a concentration of enzyme of 25 µg/mL, 100% degradation was achieved when using from 2 to 20 ppm FB1 toxin. It can be suggested that 10 µg/mL is good for low contamination around 2 ppm or lesser.

Dose of Toxins, conditions:
- 25 µg/mL, 10 µg /mL.
- 2 ppm FB1, 5 ppm FB1, 10 ppm FB1, 20 ppm FB1
- 30 mins
- pH 7.0

**Table 6. Toxin-dose degradation efficiency at two enzyme amounts.**

| ENZYME CONCENTRATION (µg/mL)¹ | TOXIN CONCENTRATION (ppm)² | DEG, % |
|---|---|---|
| 10 µg/mL | 2 | 99.3 |
| | 5 | 98.6 |
| | 10 | 98.7 |
| | 20 | 95.2 |
| 25 µg/mL | 2 | 100 |
| | 5 | 100 |
| | 10 | 100 |
| | 20 | 100 |

| | | |
|---|---|---|
| ¹Semi-pure FB1 enzyme was used. ²In the experimental solution acetonitrile (ACN) content was 8.3%, and it has negative effect on enzyme performance. So 10 ppm is the best FB1 concentration as it has up to 4% ACN and enzyme is stable. | | |

### 2.2.3. Time course of FB1 degradation

25 µg/mL of FB1 enzyme, and 2 and 10 ppm FB1 toxin were taken, and samples at 5, 10, 20 and 30 mins were taken, to evaluate degradation efficiency, at pH 7.0. As shown in Table 7, the degradation was quite fast, achieving full degradation of 2 and 10 ppm after 5 min incubation.

**Table 7. Time course of FB1 degradation.**

| Enzyme concentration (µg/mL)¹ | FB1 (ppm) | Reaction time (min) | DEG, % |
|---|---|---|---|
| 25 | 2 | 5 | 100 |
| | | 10 | 100 |
| | | 20 | 100 |
| | | 30 | 100 |
| | 10 | 5 | 100 |
| | | 10 | 100 |
| | | 20 | 100 |
| | | 30 | 100 |

| | | | |
|---|---|---|---|
| ¹Semi-pure FB1 enzyme was used. | | | |

### 2.2.4. Thermostability of FB1 enzyme

The thermostability of FB1 enzyme by performing the degradation of FB1 toxin was tested, as follows:
- Take 25 µg/mL of FB1 enzyme, incubate at 90 degrees for 3 mins.
- Then add 2, and 10 ppm FB1 toxin, and take samples at 30 mins.
- pH 7.0 buffer.
- As shown in Table 7, the degradation was quite fast, achieving full degradation of 2 and 10 ppm after 5 min incubation.

As shown in Table 8, the enzyme was quite stable after 90ºC incubation, retaining the capacity to degrade 100% of the toxins (2-10 ppm) at low dose of enzyme (25 µg/mL).

**Table 8. Thermostability of the FB1 enzyme.**

| Enzyme concentration (µg/mL)¹ | FB1 (ppm) | Reaction time (min) | DEG, % |
|---|---|---|---|
| 25 | 2 | 30 | 100 |
| 25 | 10 | 30 | 100 |

| | | | |
|---|---|---|---|
| ¹Semi-pure FB1 enzyme was used. | | | |

### 2.2.5. pH stability of FB1 enzyme

When the degradation tests were performed at different pH (3, 4, 5, 6, 7, 8) it was found almost full degradation at pH ranging from 5 to 8 (Table 9).

**Table 9. pH stability of the FB1 enzyme.**

| REACTION TIME (min) | ENZYME CONCENTRATION (µg/mL)¹ | FB1 (mg/kg) | pH | DEG, % |
|---|---|---|---|---|
| 30 | 10 | 2 | 5 | 91.9 |
| | | | 6 | 99.1 |
| | | | 7 | 100 |
| | | | 8 | 100 |

| REACTION TIME (min) | ENZYME CONCENTRATION (µg/mL)¹ | FB1 (mg/kg) | pH | DEG, % |
|---|---|---|---|---|
| 30 | 25 | 2 | 5 | 100 |
| | | | 6 | 100 |
| | | | 7 | 100 |
| | | | 8 | 100 |

| | | | | |
|---|---|---|---|---|
| ¹Semi-pure FB1 enzyme was used. | | | | |

### 2.3. FB1 assay in corn matrix

The capacity of FB1 enzyme to degrade FB1 toxin in naturally contaminated corn samples was further evaluated as follows:
- Corn grinded 1802 g/kg (FB1 and FB2) total.
- Weighed 1 g of samples added 3 mL 0.1 M phosphate buffer pH 7.0, 1:3 ratio as per Wang et al., Liquid Chromatography-Tandem Mass Spectrometry for Determination of Aflatoxin B1, Deoxynivalenol and Zearalenone in Artificial Porcine Gastrointestinal Digestive Juice, Chin J Anal Chem, 2015, 43 (1), 1-6.). carried out in triplicates.

As shown in Table 10, when the enzyme was supplied at an amount of 10-25 mg per kg naturally contaminated corn, almost full degradation after 30-90 min incubation was observed. This was observed when two different concentrations of FB1 in corn were used (Table 10).

**Table 10. Degradation efficiency in naturally contaminated corn.**

| ENZYME CONCENTRATION (mg/kg)¹ | REACTION TIME (min) | FB1 CONCENTRATION (µg/kg) | DEG, % |
|---|---|---|---|
| 10 | 30 | 1449 | 95.2 |
| | 90 | | 99.8 |
| 25 | 30 | | 99.9 |
| | 90 | | 100 |

| ENZYME CONCENTRATION (mg/kg)¹ | REACTION TIME (min) | FB2 CONCENTRATION (µg/kg) | DEG, % |
|---|---|---|---|
| 10 | 30 | 353 | 100 |
| | 90 | | 100 |
| 25 | 30 | | 100 |
| | 90 | | 100 |

| | | | |
|---|---|---|---|
| ¹Semi-pure FB1 enzyme was used. 10 mg/Kg: 1 kg/ton of feed then it will be 10x1000 mg/kg of product or 10g/Kg = 1% inclusion in MycoRaid | | | |

### 2.4. Cost of inclusion of FB1 enzyme

The efficiency of pure and semi-pure FB1 enzymes was further evaluated as follows:
- Test 5 µg/mL of each enzyme (pure or semi-pure) and 2 ppm and 10 ppm FB1; take sample at 5, 10 and 30 mins.
- Thermostability of each enzyme (pure or semi-pure), using 10 µg/mL, 2 ppm FB1 for 30 mins.
- Degradation of FB1 in corn matrix using pure or semi-pure FB1 enzymes, using 10 µg/mL and 25 µg/mL and 30 mins.

As shown in Table 11, we found that both enzyme preparations (pure or semi-pure) were highly efficient, being able to fully degrade 2 ppm toxin at a dose of 5 µg enzyme/mL after 5-30 min incubation.

**Table 11. Degradation test at 5, 10 and 30 min, using purified or semi-pure enzymes (5 µg/mL) and 2 ppm and 10 ppm FB1.**

| SAMPLE NAME | ENZYME CONCENTRATION (µg/mL) | REACTION TIME (min) | FB1 (ppm) | DEG, % |
|---|---|---|---|---|
| Semi-pure FB1 enzyme | 5 | 5 | 2 | 97.68 |
| | | 10 | | 99.57 |
| | | 30 | | 99.82 |
| | | 5 | 10 | 84.15 |
| | | 10 | | 98.35 |
| | | 30 | | 99.99 |
| Pure enzyme | 5 | 5 | 2 | 99.96 |
| | | 10 | | 100 |
| | | 30 | | 100 |
| | | 5 | 10 | 99.07 |
| | | 10 | | 100 |
| | | 30 | | 100 |

The efficiency of pure and semi-pure enzymes to degrade FB1 was also tested in naturally contaminated corn (Table 12). For that, we used 10 µg/mL and 25 µg/mL of each enzyme preparation, and 30 min incubation. As shown in Table 12, almost full or full degradation was obtained in both cases.

**Table 12. Degradation test at 30 min, when naturally contaminated corn was treated with purified or semi-pure enzymes.**

| SAMPLE NAME | ENZYME CONCENTRATION (mg/kg) | REACTION TIME (min) | FB1 (µg/kg) | DEG, % |
|---|---|---|---|---|
| Semi-pure FB1 enzyme | 10 | 30 | 28430 | 99.95 |
| | 25 | | | 100 |
| Pure enzyme | 10 | | | 99.99 |
| | 25 | | | 100 |

| SAMPLE NAME | ENZYME CONCENTRATION (mg/kg) | REACTION TIME (min) | FB2 (µg/kg) | DEG, % |
|---|---|---|---|---|
| Semi-pure FB1 enzyme | 10 | 30 | 8163 | 100 |
| | 25 | | | 100 |
| Pure enzyme | 10 | | | 100 |
| | 25 | | | 100 |

Based on the abover results, it may be necessary to add the FB1 enzyme (either pure or semi-pure) at 10-25 mg of enzyme per 1 kg Product. Using this ratio, it was finally found that FB1 toxin was fully degraded in pig gastric and intestinal juice (Table 13).

**Table 13. Degradation test in pig intestinal juice.**

| MATRIX | FB1 (ppm) | ENZYME CONCENTRATION (µg/mL)¹ | REACTION TIME (min) | DEG, % |
|---|---|---|---|---|
| PIG GASTRIC JUICE, pH 4.90 | 10 | 10 | 15 | 53.3 |
| | | | 30 | 83.7 |
| | | 25 | 15 | 83.7 |
| | | | 30 | 89.0 |
| PIG INTESTINAL JUICE, pH 5.20 | | 10 | 15 | 81.2 |
| | | | 30 | 94.4 |
| | | 25 | 15 | 98.2 |
| | | | 30 | 99.9 |

| | | | | |
|---|---|---|---|---|
| ¹Semi-pure FB1 enzyme was used. | | | | |

### 2.5. Toxin degradation test

It was further evaluated the efficiency of semi-pure FB1 enzyme for degrading toxins others than FB1, in particular, AFB1, FB2, FB3, T-2, ZEN and OTA. Test were performed as for the FB1, and the results are summarized in Table 14.

**Table 14. Degradation test of multiple toxins by FB1 hydr (semi-pure). DON= Deoxynivalenol; AFB1=Aflatoxin B1; FB1= Fumonisin B1; FB2= Fumonisin B2; FB3= Fumonisin B3; T-2= T-2 toxin; Zen= Zearalenone; OTA= Ochratoxin A.**

| Sample name | Enzyme concentration (µg/mL) | DEG %, 2ppm - 30min, pH 7, 37C | | | | | | | |
|---|---|---|---|---|---|---|---|---|---|
| | | DON | AFB1 | FB1 | FB2 | FB3 | T-2 | ZEN | OTA |
| Semi-pure FB1 enzyme | 100 | 0.1 | 0.5 | 100 | 100 | 100 | 0.6 | 0.3 | 0.2 |
| | 1000 | 0.2 | 0.56 | 100 | 100 | 100 | 0.7 | 0.4 | 0.5 |

As shown in Table 14, the enzyme efficiently degrades not only FB1, but also FB2 and FB3, whose chemical structures have been previously shown in the present document.

## Claims

1. Use of an enzyme which comprises an amino acid sequence with, at least, 80% sequence identity to sequence SEQ ID NO: 1, for fumonisin degradation in a sample.

2. The use according to claim 1, wherein the enzyme comprises the amino acid sequence SEQ ID NO: 1.

3. The use according to claim 1 or 2, wherein the fumonisin degradation comprises the degradation of, at least, one fumonisin selected from the list consisting of fumonisin B1 (FB1), fumonisin B2 (FB2), fumonisin B3 (FB3), and any combination thereof.

4. The use according to anyone of claims 1 to 3, wherein the fumonisin is FB1.

5. The use according to anyone of claims 1 to 4, wherein the sample is a raw material sample or a mixture comprising raw material.

6. The use according to claim 5, wherein the raw material is a vegetable raw material.

7. The use according to claim 6, wherein the vegetable raw material is selected from the list consisting of corn, wheat and distilled grains.

8. The use according to anyone of claims 1 to 4, wherein the sample is food or a feed product.

9. The use according to anyone of claims 1 to 4, wherein the sample is an isolated biological sample, preferably wherein the isolated biological sample is intestinal juice and/or gastric juice.

10. The use according to anyone of claims 1 to 9, wherein the enzyme concentration is from 5 µg/mL to 1000 µg/mL.

11. Method for fumonisin degradation in a sample, which comprises the following step:
a) incubating the sample with an enzyme which comprises an amino acid sequence with, at least, 80% sequence identity to sequence SEQ ID NO: 1.

12. The method according to claim 11, wherein the fumonisin degradation comprises the degradation of, at least, one fumonisin selected from the list consisting of FB1, FB2, FB3 and any combination thereof.

13. The method according to claim 11 or 12, wherein the sample is a raw material sample or a mixture comprising raw material, preferably wherein the raw material is a vegetable raw material.

14. The method according to claim 11 or 12, wherein the sample is food or a feed product.

15. The method according to claim 11 or 12, wherein the sample is an isolated biological sample.
